# EUROPEAN PATENT SPECIFICATION

(11) **EP 0 407 056 B1**
(45) Date of publication and mention of the grant of the patent: **27.08.1997**
(21) Application number: 90306696.7
(22) Date of filing: 19.06.1990
(51) Int. Cl.: A61L 15/07, D06M 23/08, D06M 15/564, C09D 175/04

(54) **Curable resins with reduced foaming characteristics and articles incorporating same**
Härtbare Harze mit verminderter Schaumbildung und diese Harze enthaltende Gegenstände
Résines peu moussantes et articles contenant ces résines

(30) Priority: 07.07.1989 US 376421
(43) Date of publication of application: 09.01.1991
(73) Proprietor: MINNESOTA MINING AND MANUFACTURING COMPANY, St. Paul, Minnesota 55133-3427 (US)
(72) Inventor: Polta, Charles C., c/o Minnesota Mining and, St. Paul, Minnesota 55133-3427 (US); Scholz, Matthew T., c/o Minnesota Mining and, St. Paul, Minnesota 55133-3427 (US)
(74) Representative: Baillie, Iain Cameron

(56) References cited:
- EP-A- 0 221 669

## Description

### Background

### Field of the Invention

The present invention relates to water-curable resins, and in particular, to orthopedic casting materials incorporating such curable resins.

### The Prior Art

Many different orthopedic casting materials have been developed for use in the immobilization of broken or otherwise injured body parts. One of the first casting materials developed for this purpose was plaster of Paris bandage.

More recently, water-curable, isocyanate-functional, polyurethane prepolymers were found to be extremely useful in formulating a resin for orthopedic casting materials, as disclosed, for example, in U.S. Patent No. 4,502,479 (Garwood et al.), U.S. Patent No. 4,609,578 (Reed), U.S. Patent No. 4,667,661 (Scholz et al.), and U.S. Patent No. 4,774,937 (Scholz et al.). Most commonly, a knitted fiberglass fabric is used as the scrim in combination with the resin.

To initiate the cure of such water curable orthopedic casting materials, the material is contacted with water, typically by immersing a roll of the material in water. Upon immersion, the curing process begins as the isocyanate-functional groups begin polymerizing in the presence of the water. Such polymerization is often aided or controlled by the use of a catalyst, such as is disclosed, for example, in U.S. Patent No. 4,705,840 (Buckanin). During this curing process, carbon dioxide is formed and released from the resin in the form of bubbles. Unfortunately, the evolution of carbon dioxide bubbles, if not properly controlled, can result in undesirable foaming.

Foaming is undesirable from several standpoints. First, after a roll of orthopedic casting tape has been applied, foaming can cause the end of the tape to percolate up and away from the formed cast. Second, foaming is undesirable because it can decrease layer to layer lamination and hence the strength and durability of the resultant cured orthopedic casting material. Third, foaming can adversely affect the handling characteristics of the material, and can result in undesirable dripping.

European Document EP-A-0 355 635 deals with an orthopaedic casting material having reduced foaming, reduced tack and reduced slip properties. This material comprises a fabric that is impregnated and/or coated with polyisocyanate prepolymer having incorporated therein an oil and a polymeric micropowder of a flourocarbon polymer or polyethylene wax.

Several antifoaming agents, e.g., DB-100 silicone fluid (Dow Corning) and silicone Antifoam A (Dow Corning) have been used in the art to suppress foaming.

The time from initial activation of the material to the time that the material has cured to the point that significant immobilization has been achieved is known as the set time. Typically, a set time from about 2 to 18 minutes following activation of the resin is desirable, and a set time of from about 3 to 5 minutes is most preferred for most orthopedic applications. As mentioned previously, a catalyst can be added to the resin system to control the rate of cure, and thus control the set time. One approach used in the art to decrease the set time is to increase the quantity of catalyst. Others have sought to decrease the set time of an orthopedic casting material by providing for faster water penetration into the resin by including hydrophilic constituents in the resin.

### Brief Summary of the Invention

In general, the invention features an article that includes a fabric scrim (e.g., knitted fiberglass) coated with a water-curable an isocyanate-functional polyurethane prepolymer containing a stable dispersion of hydrophobic polymeric particles. Stable dispersion, as used herein, means a dispersion in which the polymeric particles remain substantially suspended and do not form macroscopic aggregates, nor do the particles separate from the resin either by floatation or sedimentation. Hydrophobic, as used herein, refers to a material that does not readily dissolve in water or appreciably swell when exposed to water.

In some preferred embodiments, the polymeric particles reduce foaming of the resin during cure. When the article is a casting material, reducing foaming results in improved lamination, which is often not pronounced at the end of the material, stronger and more durable casts, improved handling characteristics (e.q., less dripping), and improved porosity.

In other preferred embodiments, the polymeric particles decrease the set time of the articles. The object to which the article is applied is thus immobilized in less time.

In other preferred embodiments, the polymeric particles increase the wet strength of the article after cure. Preferably, the warm wet ring strength of the material, after cure, is increased by at least 25%.

In other preferred embodiments, the polymeric particles have an average diameter of less than about 20 microns (preferably about 0.01 micron to about 20 microns, more preferably about 0.1 micron to about 10 microns, most preferably 0.3 micron to about 5 microns). Particles of this size generally provide the decreased foaming, decreased set time, and better wet strength.

In other preferred embodiments, the resin includes between about 0.5% and 10% (more preferably 1% and 6%, most preferably 2% and 4%) of the polymeric particles by weight.

The preferred polymeric particles are those made from a vinyl monomer for example polyacrylonitrile or a copolymer of acrylonitrile and styrene; polyurea; epoxy resin based polymer or combinations thereof.

The invention features, in another aspect, a method of applying a casting material which has a resin containing the stable dispersion of the hydrophobic polymeric particles.

Other features and advantages of the invention will be apparent from the description of the preferred embodiment thereof, and from the claims.

### Detailed Description of the Preferred Embodiments

The preferred articles exhibit reduced foaming, faster set times, and increased wet strength, in particular in warm water, herein referred to as "warm wet strength", after curing. These articles have particular utility when used in orthopedic cast applications; however, such is not their only utility. Hence, although the following discussion is set forth in the context of an orthopedic casting material application, it will be appreciated that the applications and uses of the articles of the present invention are not so limited, and a few of the other utilities of the present invention will be noted hereinafter.

The curable resins of the present invention are those resins which employ a water phase during curing where the benefits of the present invention can be realized. Hence, the curable resins of the present invention are water-curable, isocyanate-functional, polyurethane prepolymers.

Presently preferred resins for use in the present invention include those disclosed in U.S. Patent No. 4,667,661 (Scholz et al.) and U.S. Patent No. 4,774,937 (Scholz et al.), which patents are incorporated herein by reference. The polyurethane prepolymer resins disclosed in these patents are formed by the reaction of a polyol with an excess of a polyisocyanate. The resins disclosed in the two aforementioned patents also include tack reducing agents which facilitate application of the orthopedic casting materials. However, in the present invention, these resins are modified by using a polyol (which represents at least a part of the total polyol concentration employed) having a stable dispersion of hydrophobic polymeric particles. The particles serve to: 1) reduce foaming during cure, 2) decrease the set time, and/or, 3) result in cured materials having greater strength, in particular, greater warm wet strength. Preferred embodiments of the present invention provide one or more of the foregoing benefits, while the most preferred embodiments provide all three of the foregoing benefits.

Preferably, the hydrophobic polymeric particles are made from vinyl monomers. However, any monomers may be employed which will form hydrophobic polymeric particles that remain as a stable dispersion in the resin. Examples of polymeric particles which have been found suitable for purposes of the present invention are those made of polyacrylonitrile, a copolymer of acrylonitrile and styrene, and polyurea (formed, for example, from toluene diisocyanate and ethylenediamine). Polymer particle containing polyols made from epoxy based resins or combinations of any of the foregoing may also be used to form the polymeric particles used in the present invention.

Several polyols are commercially available which already have such polymeric particles dispersed therein, and are thus suitable for practicing the present invention. For example, "Niax" E-562 polyol (available from Union Carbide Corporation, Wheeling, East Virginia) which contains polymeric particles made of a copolymer of acrylonitrile and styrene (in a 50/50 weight % ratio); Niax E-701 polyol (also available from Union Carbide Corporation) which contains polymeric particles made of polyacrylonitrile; and Multranol 9151 polyol (available from Mobay Chemical Corporation, Pittsburgh, Pennsylvania) which contains polymeric particles made of polyurea; have been found to be useful in practicing the present invention. The presently most preferred polyols having polymeric particles dispersed therein are the aforementioned Niax E-562 and Niax E-701 polyols, which are referred to by Union Carbide Corporation as "Niax Performance Polyether Polymer Polyols." It will be appreciated that Niax polymer polyols other than Niax E-562 and Niax E-701 may be useful in the present invention, and that particle containing polyols other than the group described hereinabove may also fall within the scope of the present invention. Hence, the foregoing list should not be considered in any way as limiting or comprehensive, but only as illustrative.

The preparation of polymeric particle-containing polyols is described in United States Patent Nos. 3,304,272; 4,125,505; and 3,383,351, all of which are hereby incorporated by reference herein.

Without being bound to a particular theory, it is presently believed that the polymeric particles used in accordance with the invention form a stable dispersion in the resin through an interaction with the polyol used in the resin. More specifically, it is felt the polyol molecules sterically stabilize the particles so that macroscopic aggregation, sedimentation or floatation of the particles in the resin does not occur. Steric stabilization is a conventional term of art used to describe the stabilization of colloidal particles by absorbed nonionic macromolecules; a more detailed description can be found in Kirk-Othmer, Encyclopedia of Chemical Technology, at 364-66 (3d. ed., John Wiley and Sons, NY). It is believed that such stabilization results when the monomers that form the polymer particles are polymerized in the polyols that are contained in typical polyurethane resins. In this sense, the polyol is a reactive medium. The hydroxyl groups on these polyols, particularly the primary hydroxyl groups, appear to cause a grafting of the polyol to the polymer particles, which results in the particles being sterically stabilized.

For reasons not presently understood, the size of the polymeric particles appears to be important in the practice of the present invention. Preferably, the average diameter of the particle should be less than about 20 microns. There apparently is no minimum average diameter of particle needed to achieve the benefits of the invention, and it is believed that smaller particle sizes work best. Preferably, the average diameter of the particles is greater than 0.01 micron. More preferably, the average diameter is between about 0.1 micron and 10 microns, and the most preferred particles have an average diameter of between about 0.3 micron and about 5 microns. Although polymeric particle sizes greater than the foregoing may be employed, it is believed that some of the benefits of the polymeric particle may be sacrificed if the particle size is too great.

In the commercially available polyols mentioned herein, the polymeric particles comprise from about 20% to about 38% by weight of the polyol. It is believed that polyols containing 10% to 45% by weight function adequately. When incorporating such polyols into the curable resins of the present invention, the polymeric particles preferably comprise from about 0.5% to about 10% by weight of the resin, more preferably from about 1% to about 6% by weight of the resin, and most preferably from about 2% to about 4% by weight of the resin.

As mentioned, the curable resins of the present invention are formed, for example, in accordance with the teachings of U.S. Patent No. 4,667,661 and U.S. Patent No. 4,774,937, with the modification that a polyol containing a stable dispersion of hydrophobic polymeric particles as disclosed herein is also employed. It may also be desirable to vary the proportions of the ingredients in the resin somewhat from the teachings of U.S. Patent Nos. 4,667,661 and 4,774,937 when using polymeric particle containing polyols in accordance with the present invention. For example, it may be desirable to slightly increase the ratio of isocyanate equivalents to polyol equivalents and to slightly decrease the isocyanate equivalent weight of the resin. Furthermore, because of the faster set times achieved in the present invention, the amount of catalyst employed can be desirably decreased.

In deciding whether or not to so vary the relative proportions of the resin ingredients, the following criteria should be considered: 1) proper ring strength and ring delamination strength of the resultant cast should be preserved by ensuring sufficient resin content, while at the same time preserving adequate porosity; 2) the viscosity of the resin should be kept within a range that enables the resin-coated casting material to be easily unwound and applied to the patient, generally within the range of 10,000 to 300,000 centipoise when measured on a Brookfield RVT Viscometer using spindles #6 or #7, more preferably within the range of 10,000 to 100,000 centipoise, and most preferably within the range of 10,000 to 80,000 centipoise; 3) the set time of the material should be from about 2 to about 18 minutes, more preferably from about 2.5 to about 10 minutes, and most preferably from about 3 to about 5 minutes.

In accordance with the teachings of U.S. Patent Nos. 4,667,661 and 4,774,937, the curable resins used in the present invention are polymerizable to a thermoset state. The resin is preferably nontoxic in the sense that it does not give off significant amounts of toxic vapors during curing which may be harmful to either the patient or the person applying the orthopedic casting material, and also in the sense that it does not cause skin irritation either by chemical irritation or by the generation of excessive heat during cure. Furthermore, the resin must be sufficiently reactive with water to ensure rapid hardening of the orthopedic casting material once it has been applied, but not so reactive that it does not allow for sufficient working time to apply and shape the orthopedic cast or splint. Initially, the orthopedic casting material must be pliable and conformable and should adhere to itself. Then in a short time following the completion of application, it should become rigid, or at least semi-rigid, and strong enough to support the loads and stresses to which the cast of splint is subjected by the activities of the wearer. Thus, the orthopedic casting material must undergo a change of state from a flexible condition to a relatively rigid condition in a matter of minutes.

As mentioned, presently preferred resins used in the present invention are water-curable, isocyanate-functional, polyurethane prepolymer resins. These resins are formed by reacting a polyol with an excess of a suitable polyisocyanate. At least a portion of the total amount of polyol employed contains polymeric particle containing polyols disclosed herein. However, in many of the preferred embodiments of the present invention, the total polyol concentration is represented both by polyols which contain polymeric particles as disclosed herein and polyols which contain no such polymeric particles. Indeed, such a "blend" of polymeric particle containing polyols and conventional polyols is presently preferred in most embodiments.

Conventional polyols containing no polymeric particles which may be used as part of the total polyol concentration in forming the polyurethane prepolymers of the present invention include polypropylene ether glycols (available from Union Carbide, Danbury, Connecticut as Niax™ PPG and from BASF Wyandotte Corp., Parsippany, New Jersey as Pluracol™ P), polybutylene ether glycols (available from Dow Chemical, Midland, Michigan as XAS 10961.00 experimental polyol), polytetramethylene ether glycols (available from Quaker Chemical Company, Conshohocken, Pennsylvania as Polymeg™), polycaprolactone diols (available from Union Carbide as the Niax™ PCP series of polyols), and polyester polyols (hydroxyl terminated polyesters obtained from the esterification of dicarboxylic acids and diols such as the Lexorez™ polyols available from Inolex Corp., Chemical Division, Philadelphia, Pennsylvania). As will be appreciated by those skilled in the art, the rigidity of the cured resin can be reduced by increasing the molecular weight of the polyols.

It will be understood that, as used herein, the term "polyol" also includes virtually any functional compound having active hydrogen in accordance with the well-known Zerevitinov test, as described, for example, in Chemistry of Organic Compounds by Carl R. Noller, Chapter 6, pp. 121-122 (1957). Thus for example, thiols and polyamines could also be used as "polyols" in the present invention, and the term "polyols" will be considered to include such other active hydrogen compounds.

In choosing an appropriate polyisocyanate, it is presently preferred to use an isocyanate which has a relatively low volatility, such as diphenylmethane diisocyanate (MDI), rather than a more volatile material such as toluene diisocyanate (TDI), which is also more toxic. Presently preferred isocyanates include 4,4-diphenylmethane diisocyanate, 2,4′-diphenylmethane diisocyanate, and mixtures of these isomers together with possible small quantities of 2,2′-diphenylmethane diisocyanate (typical of commercially available diphenylmethane diisocyanate). However, isocyanates such as aromatic polyisocyanates and their mixtures which are derived from phosgenation of the condensation product of aniline and formaldehyde may also be used.

One example of a presently preferred resin which may be used in the present invention involves the reaction of an isocyanate known as Isonate™ 2143L (a mixture containing about 73% MDI) which is available from the Dow Chemical Co., with a mixture of polypropylene oxide polyols which are available from Union Carbide and are known as Niax™ PPG 2025 and Niax™ LG-650, in addition to one of the polymeric particle containing polyols disclosed herein such as Niax E-562 polyol. To prolong the shelf-life of the resin, it is also preferable to include from about 0.01% to about 1% by weight of benzoyl chloride or other suitable stabilizer.

The reactivity of the curable resin, once it is exposed to the water, can be controlled by the use of a proper catalyst. The reactivity must not be so great that: (1) a hard film quickly forms on the resin surface preventing further penetration of the water into the bulk of the resin, or (2) the cast or splint becomes rigid before the application and shaping thereof has been completed. To produce suitable orthopedic casts and splints in accordance with the present invention, a set time of from about 2 to about 18 minutes following activation of the curable resin is preferred, with a more preferable set time being from about 2.5 to about 10 minutes, and a most preferable set time being from about 3 to about 5 minutes. Thus, the curable resins of the present invention also preferably contain a catalyst to control the set time and cure time of the resin.

Suitable catalysts for moisture curing polyurethane prepolymer resin systems are well-known. For example, tertiary amine catalysts such as 4-[2-[1-methyl-2-(4-morpholinyl)ethoxy]ethyl]morpholine (MEMPE) described in commonly assigned, U.S. Patent. No. 4,705,840 (Buckanin), in amounts ranging from about 0.5% to about 5% by weight of the resin system, may be used for this purpose. The MEMPE catalyst disclosed in U.S. Patent No. 4,705,840, which patent is incorporated herein by reference, is the presently preferred catalyst system for use in connection with the present invention.

Foaming of the resin should be minimized. In addition to the hydrophobic polymeric particles, the preferred resins may include a foam suppressor such as DB 100 silicone fluid (Dow Corning), silicone Antifoam A (Dow Corning, Midland, Michigan), or silicone surfactant L550 or L5303 (available from Union Carbide). It is presently preferred to use the Dow Corning DB-100 silicone fluid at a concentration of about 0.1% to about 1% by weight of the resin.

As mentioned, it is also preferred to make the curable resin of the present invention less tacky in accordance with the invention described in commonly assigned, U.S. Patent No. 4,667,661 (Scholz et al.) and U.S. Patent No. 4,774,937 (Scholz et al.). Reduced tackiness may be achieved by a number of means as described in U.S. Patent No. 4,667,661 and U.S. Patent No. 4,774,937. One technique for achieving such tack reduction is to lightly coat the surfaces of the resin-coated scrim with a mixture of a polydimethylsiloxane, having a viscosity of at least about 100 centistokes, and polyethylene oxide long chain aliphatic hydrocarbon waxes. Alternatively, a small amount of a polyethylene oxide - polypropylene oxide block copolymer (such as Pluronic F-108 available from BASF Wyandotte) may be added to the resin during prepolymer preparation, after which the polydimethyl-siloxane may be applied onto the surface of the orthopedic article as before. The polydimethylsiloxane reduces resin tackiness prior to contact with water. The hydrophilic polyethylene oxide materials provide additional tack reduction upon contact with water.

The preparation of the orthopedic casting materials of the present invention generally involves coating the curable resin onto the fabric scrim by standard techniques. Generally, the scrim should be resin loaded to the point where the resin represents from about 35% to about 80% by weight of the total weight of the resin-coated scrim. In the case of a fiberglass scrim, the resin preferably represents from about 35% to about 60% by weight of the total weight of the resin-coated scrim, and preferably from about 38% to about 45% by weight. Manual or mechanical manipulation of the resin (such as by a nip roller or wiper blade) into the scrim is usually not necessary. However, some manipulation of the resin into the fabric may sometimes be desirable to achieve proper impregnation. Care should be given not to stretch the fabric scrim during resin coating, however, so as to preserve the stretchability of the material for its later application around the desired body part.

The curable resins of the present invention may be used with a variety of well-known scrims for use as orthopedic casting materials or for other applications. Although many materials are well known for this purpose, fiberglass is presently preferred. In this regard, in one presently preferred embodiment of the present invention, the scrim comprises an extensible, heat-set, knitted fiberglass fabric as set forth in U.S. Patent No. 4,609,578 (Reed), which patent is incorporated herein by reference. One example of a knitted fiberglass scrim which is within the scope of U.S. Patent No. 4,609,578 is known by 3M, St. Paul, Minnesota, as the Scotchcast® 2 knitted fiberglass scrim. The Scotchcast® 2 scrim is used in the manufacture of 3M's Scotchcast® 2 and Scotchcast® Plus orthopedic casting materials.

In one presently preferred embodiment, a series of projections is also formed along such a knitted fiberglass scrim in order to enhance the lamination properties thereof. A detailed description of scrims having such projections and the enhanced lamination achieved thereby is disclosed in U.S. patent application Serial No. 376,873, filed on the same date as the present application, filed in the names of Matthew T. Scholz, Robert L. Assell, Ralph A. Wilkens, and Charles E. Alexson, under Express Mailing Certificate No. B75869812, and entitled "Orthopedic Casting Materials Having Superior Lamination Characteristics and Methods for Preparing Same"; such patent application is incorporated herein by reference. Such projections can be formed by abrading the scrim with, for example, a knurled roller, a knife edge, sharp of blunt teeth, or by other techniques.

As disclosed in the aforementioned concurrently filed patent application, a projection is considered to be a filament bundle which serves to enhance lamination and typically has 8 or more filaments per bundle. As further set forth in that concurrently filed patent application, the fabric scrims preferably have from about 75 to about 1,500 projections per gram of fabric scrim on the average, more preferably from about 100 to about 1,000 projections per gram of fabric scrim, and most preferably from about 300 to about 700 projections per gram of fabric scrim.

By using the improved curable resins of the present invention with the improved fabric scrims of the above-mentioned concurrently filed patent application, the benefits of both the improved scrim and the improved resin can be realized in a single resin-coated-material, whether used for orthopedic purposes or for other applications. Indeed, such a combination constitutes one of the presently most preferred embodiments.

Orthopedic casting materials prepared in accordance with the present invention are applied to humans or other animals in the same fashion as other known orthopedic casting materials. First, the body member or part to be immobilized is preferably covered with a conventional cast padding and/or stockinet to protect the body part. Next, the curable resin is activated by dipping the orthopedic casting material in water. Excess water may then be squeezed out of the orthopedic casting material, and the material is wrapped or otherwise positioned around the body part so as to properly conform thereto. Preferably, the material is then molded and smoothed to form the best fit possible and to properly secure the body part in the desired position. Although often not necessary, if desired, the orthopedic casting materials may be held in place during cure by wrapping an elastic bandage or other securing means around the curing orthopedic casting material. When curing is complete, the body part is properly immobilized within the orthopedic cast or splint which is formed.

The preferred casting materials of the present invention exhibit reduced foaming, decreased set time, and improved wet strength, particularly warm wet strength.

Foaming can occur in at least two different regions of the orthopedic casting system: the resin phase and the aqueous or water phase. The resin phase is represented by the resin on the scrim which is activated when contacted with water. The water phase is represented by the water which contacts or is otherwise associated with the resin during cure. As the carbon dioxide bubbles are released during cure, they pass both through the resin phase and through the water phase; thus foaming can occur in either or both of these phases. Foaming in the aqueous phase is often clearly visible, as "suds", during cure. Foaming in the resin phase often is not clearly discernible visibly. The reduced foaming benefits of the present invention result primarily from the foam-suppressive effect of the hydrophobic polymeric particles on the aqueous phase.

The presence of the hydrophobic polymeric particles preferably decrease set time by at least 5%, more preferably by at least 10%, and even by up to 60% or more. The decreased set time achieved through use of the particles means that less catalyst can be used to achieve a comparable set time to casting materials that do not include the polymeric particles.

The hydrophobic polymeric particles preferably increase the warm wet strength of the casting material by at least 25%, and even by up to 100% or more. It is believed the increased wet strength is a result of the hydrophobicity of the particles, which makes the overall system less water absorptive.

Summarized below are tests which were used to determine "foam reduction," "early strength" (which is related to set time), and "warm wet strength." Hence, whenever the terms "foam reduction" (or reduced foaming), "early strength", and "wet strength" (also sometimes referred to as "warm wet ring strength"), it will be understood that these terms refer to the tests set forth herein below. It is understood that to determine if a reduction in foaming, decrease in set time, or increase in wet strength is being achieved with a particular article, the values obtained should be compared to an article that does not contain the stable dispersion of hydrophobic polymeric particles. In making the comparison, however, the NCO/OH (total isocyanate group equivalent to total hydroxyl group equivalent) ratio and isocyanate (NCO) equivalent weight (g resin/equivalence of unreacted isocyanate) of the examples should be approximately the same. Persons skilled in the art know how to make the particular adjustments; some of the samples below are also illustrative.

### Foam Reduction Test

A 12 foot long resin-coated material is taken out of a pouch, equilibrated to 20°C, and immersed completely in deionized water having a temperature of about 80°F (27°C) for about 30 seconds. In order to observe the foaming of the material during cure, the article is applied around a 2 inch (5.08 centimeter) diameter, 12 inch (30.5 centimeter) long mandrel (covered with a thin layer of polyester stockinet such as 3M Synthetic Stockinet MS02) in three overlapping layers. The wrapping around the mandrel is achieved within about 45 seconds from the time the material is immersed. After application around the mandrel, the material is aggressively rubbed by hand over its entire length until two minutes has elapsed from the time the pouch is first opened. Visual inspection shows clearly the qualitative degree of foaming, particularly in the water phase, and the approximate duration of the foaming. In this regard, the peak amount of foaming can be estimated in teaspoons.

### Early Strength Test (Set Time Test)

This test measures the ring strength of a resin-coated material 5 minutes after cure had been initiated. The so-called "early strength" of a material measured 5 minutes into the cure gives an indication as to just how fast the material is curing, and hence, is directly related to the set time. In other words, higher early strengths indicate faster set times and vice versa.

In this test, a cylindrical ring comprising 6 layers of the resin-coated material is wrapped around a stockinet covered (such as with 3M Synthetic Stockinet MS02) mandrel having a diameter of 2 inches (5.04 centimeters) under a tension of 0.25 pounds per inch width of material (0.045 kg/cm). The width of the ring is equal to the width of the material used (preferably, 3-4 inches).

After wrapping the resin-coated material around the mandrel, the cylindrical ring on the mandrel is submerged in deionized water for about 30 seconds at about 25°C without agitation. After submersion, the mandrel and cylindrical ring sample are removed from water. Four minutes after initial immersion the rings are removed from the mandrel. Five minutes after the initial immersion of the cylindrical ring in the water, the ring strength of the cylindrical sample is determined using an Instron Model 1122 instrument.

The ring strength at 5 minutes (or "early strength") is determined by placing the cylinder in the fixture mounted to the Instron instrument. Compression loads are then applied to the cylindrical ring sample along its exterior and parallel to its axis. The cylindrical ring is placed lengthwise between the two bottom bars of the fixture (the bars being 1.9 centimeters wide, 1.3 centimeters in height, and 15.2 centimeters long), with the bars spaced about 4 centimeters apart. The inside edges of the bars are machined to form a curved surface having a 1/8 inch (0.31 centimeter) radius. A third bar (0.63 centimeters wide, 2.5 centimeters high, and 15.2 centimeters long) is then centered over the top of the cylinder, also parallel to its axis. The bottom or contacting edge of the third bar is machined to form a curved surface having a 1/8 inch (0.31 centimeter) radius. The third bar is brought down to bear against and crush the cylinder at a speed of about 5 cm/min. The maximum or peak force which is applied while crushing the cylinder is then recorded as the ring strength, which in this particular instance is the "early strength" (expressed in terms of force per unit length of the cylinder, i.e., newtons/cm). For each resin-coated material, 5 samples are tested, and the average peak force applied is then calculated.

### Warm Wet Strength Test

In this test, the "warm wet strength" of certain cured cylindrical ring samples of the resin-coated materials of the present invention is determined. Each cylindrical ring comprises 6 layers of the resin-coated material having an inner diameter of 2 inches (5.08 centimeters). The width of the ring formed is the same as the width of the resin-coated material employed.

Each cylindrical ring is formed by taking a roll of the resin-coated material from its storage pouch and immersing the roll completely in deionized water having a temperature of about 80°F (27°C) for about 30 seconds. The roll of resin-coated material is then removed from the water and the material is wrapped around a 2 inch (5.08 centimeter) mandrel, covered with a thin layer of stockinet such as 3M Synthetic Stockinet MS02, to form 6 complete uniform layers using a controlled wrapping tension of about 45 grams per centimeter width of the material. Each cylinder is completely wound within 30 seconds after its removal from the water.

After 30 minutes from the initial immersion in water, the cured cylinder is removed from the mandrel, and allowed to cure for 24 hours in a controlled atmosphere of 75°F ± 3°F (34°C ± 2°C) and 55% ± 5% relative humidity. After this time, each cylinder is then immersed in water at about 113°F (45°C) for about 30 minutes, removed from this warm water bath, and immediately placed in the Instron instrument fixture for testing.

Once in the instrument fixture, compression loads are applied to the cylindrical ring sample along its exterior and parallel to its axis, using the same Instron instrument fixture and procedure as for the early strength test above. Again, the cylinder is crushed at a speed of about 5 cm/min. The maximum or peak force which is applied while crushing the cylinder is then recorded as the ring strength, which in this particular instance is the "warm wet strength" (expressed in terms of force per unit length of the cylinder). For each material, at least five samples are tested and the average peak force applied is then calculated.

The following are examples of some preferred casting materials. For some examples, a comparative example is also provided. However, these examples are given for the purpose of illustration only, and should not be considered comprehensive or restrictive.

### Example 1 and Comparative Example A

In Example 1, a water-curable resin and resin-coated material within the scope of the present invention were prepared. In Example 1, a polyol containing polymeric particles (made of polyacrylontrile) within the scope of the present invention (Niax E-701) was employed. For purposes of comparison, a control resin was also prepared in Comparative Example A which contained no Niax E-701 such that the resulting resin had no polymeric particles. The NCO/OH ratio of Example 1 and Comparative Example A was 3.15, and the NCO equivalent weight of both was 375 g/equiv. NCO.

An apparatus was first assembled including a 3.8 liter (1 gallon) glass vessel equipped with a 12.7 centimeter x 2.54 centimeter x 0.318 centimeter (5 inches x 1 inch x 1/8 inch) Teflon™ impeller, an addition funnel, a nitrogen purge line, and a thermometer. This reaction vessel was purged with nitrogen for 15 minutes to ensure that the apparatus was relatively dry. The following chemical ingredients listed in Table I below were added to the reaction vessel through the addition funnel in the exact order indicated in Table I, with each successive ingredient being added at five minute intervals. (In other words, 1975.4 grams of Isonate 2143L were added first to the reaction vessel, five minutes later 1.75 grams of benzoyl chloride were added, five minutes later 140.18 grams of Pluronic F-108 were added, and so on.)

**TABLE I**

| Ingredient | Example 1 | | Comparative Example A | |
|---|---|---|---|---|
| | Wt (g) | Wt % | Wt (g) | Wt % |
| Isonate 2143L isocyanate (Dow Chemical Co.) | 1975.98 | 56.46 | 1975.4 | 56.44 |
| Benzoyl Chloride | 1.75 | 0.05 | 1.75 | 0.05 |
| Pluronic F-108 polyethylene oxide -polypropylene oxide block copolymer (BASF) | 139.99 | 4.00 | 140.18 | 4.00 |
| DB-100 silicone fluid (Dow Corning) | 6.30 | 0.18 | 6.31 | 0.18 |
| Butylated hydroxy toluene (BHT) | 16.80 | 0.48 | 16.21 | 0.48 |
| PPG-425 polyol (Union Carbide) | 589.31 | 16.84 | 424.5 | 12.13 |
| PPG-725 polyol (Union Carbide) | 583.68 | 16.68 | 888.66 | 25.39 |
| MEMPE Catalyst | 46.20 | 1.32 | 46.24 | 1.32 |
| Niax E-701 polyol containing polymeric particles (Union Carbide) | 139.99 | 4.00 | 0 | 0 |

As the viscosity of the reaction mixture increased, the agitation rate was gradually increased from a starting rate of about 50 RPM to a finishing rate of about 100 to 150 RPM. Insulation had also been placed around the reaction vessel such that the exotherm created by the reactive ingredients raised the temperature within the reaction vessel from about 45°C to about 60°C during the procedure. After all ingredients had been included in the reaction mixture, the insulation was removed along with the agitator, thermometer, and addition funnel. The reaction vessel was then sealed, and the resultant resin was allowed to cool for about 24 hours.

After cooling, the resins formed in Example 1 and Comparative Example A were separately coated onto the 3 inch wide knitted fiberglass scrim known by 3M as the Scotchcast® 2 scrim. (This scrim is within the scope of United States Patent No. 4,609,578.) In each of Example 1 and Comparative Example A, the resin was coated onto the fiberglass scrim in an amount such that the resin represented about 42.5% by weight of the resultant resin-coated material. The resin-coated tape was then cut into 3.66 meter (12 feet) lengths, and each length of resin-coated material was wound around a 1.9 centimeter diameter polyethylene plastic core. The individual rolls of resin-coated material were then individually packaged in moisture-proof pouches.

Each of the individually packaged rolls of resin-coated tape were allowed to age for about two weeks at about 4°C. After this two-week period, each roll of tape was removed from its respective pouch, warmed to about 20°C, and the cure was initiated by immersing each roll of tape in water. After immersion, each tape was applied around a 2 inch (5.08 centimeter) diameter by 12 inch (30.5 centimeter) long mandrel in multiple overlapping layers, and the tape was aggressively rubbed by hand over the entire outer surface. By so wrapping the tapes around the mandrels, foaming of the tapes could be more carefully observed. In this regard, the resin-coated tapes of Example 1 were observed to foam considerably less than the resin-coated tapes of Comparative Example A; reduced foaming was visibly observed in the water phase.

Another set of resin-coated tapes prepared in accordance with Example 1 and Comparative Example A were allowed to age for six months at 25°C in their respective pouches. The resin-coated tapes were removed at the end of the six-month period and were cured around mandrels in accordance with the procedure set forth above. Again, reduced foaming in the aqueous phase visibly was observed for the resin-coated tapes of Example 1 over the resin-coated tapes of Comparative Example A.

### Example 2

In this Example 2, a water-curable resin and resin-coated material within the scope of the present invention were prepared following the same procedure as set forth for Example 1 above, except that a different polymeric particle containing polyol was employed (Niax E 562), different nonparticle containing polyols were employed (LG 650 and PPG-2025 polyols), and the relative proportions of the ingredients were somewhat different.

The resin of Example 2 was prepared in accordance with the procedure of Example 1 using the ingredients and amounts are set forth in Table II below, the ingredients having been added in the order indicated in Table II.

**TABLE II**

| Ingredient | Example 2 | |
|---|---|---|
| | Wt (q) | Wt % |
| Isonate 2143L | 1770.00 | 59.00 |
| Benzoyl Chloride | 1.49 | 0.05 |
| Pluronic F-108 | 120.17 | 4.00 |
| DB-100 | 5.38 | 0.18 |
| BHT | 14.34 | 0.48 |
| LG-650 polyol (Union Carbide) | 186.00 | 6.20 |
| PPG-2025 polyol (Union Carbide) | 503.10 | 16.77 |
| MEMPE | 39.62 | 1.32 |
| Niax E-562 polyol containing polymeric particles (Union Carbide) | 360.00 | 12.00 |

The prepared resin was coated onto the same type of fiberglass scrim and in the same fashion as for Example 1, and individual rolls of the resin-coated material were formed and individually packaged as in Example 1.

The warm wet strength test was performed on five rolls of the resin-coated tape in accordance with the test set forth in the specification. The average warm wet strength was 63 newtons/cm.

### Examples 3 and 4

In Examples 3 and 4, Niax E-562 polymeric particle containing polyol and Niax E-701 polymeric particle containing polyol, respectively, were employed. Water curable resins were prepared and coated onto fiberglass scrims in the same fashion as Example 1, using the ingredients and amounts shown in Table III below, the ingredients having been added in the order indicated in Table III.

**TABLE III**

| Ingredient | Weight in Grams | | Weight % | |
|---|---|---|---|---|
| | Ex. 3 | Ex. 4 | Ex. 3 | Ex. 4 |
| Isonate 2143L | 33937 | 2086.3 | 57.5 | 56.4 |
| Para-toluene-sulfonyl chloride | 36.3 | 1.85 | 0.06 | 0.05 |
| DB-100 | 104.4 | 16.66 | 0.18 | 0.18 |
| BHT | 281 | 17.76 | 0.48 | 0.48 |
| Pluronic F-108 | 2361 | 148 | 4.0 | 4.0 |
| MEMPE (Catalyst) | 681 | 48.8 | 1.32 | 1.32 |
| PPG-2025 | 12412 | 0 | 21.03 | 0 |
| LG-650 | 3396 | 273.5 | 5.75 | 7.4 |
| Niax E-562 | 5816 | 0 | 9.85 | 0 |
| Niax E-701 | 0 | 1117 | 0 | 30.02 |

The resins were coated onto fiberglass scrims, cut into individual rolls, and packaged in moisture-proof packages in accordance with the procedure of Example 1.

### Examples 5-10 and Comparative Examples B and C

In Examples 5-10, the improved early strengths of certain resin-coated materials within the scope of the present invention were investigated. In each of Examples 5-10 and Comparative Examples B and C, resin coated tapes were made, cut into individual rolls, and packaged in accordance with the procedure of Example 1, using the following ingredients and amounts set forth in Tables IV-XI below, the ingredients being added to the resin mixture in the order indicated in the Tables. The NCO/OH ratio for Examples 5-7 and Comparative Example B was 3.45 and the NCO equivalent weight was 375 g/equiv. NCO. The NCO/OH ratio for Examples 8-10 and Comparative Example C was 4.27, and the NCO equivalent weight was 333 g/equiv. NCO.

**TABLE IV**

| (Comparative Example B) | | |
|---|---|---|
| Ingredient | Wt % | Grams |
| Isonate 2143L | 54.45 | 2014.65 |
| Benzoyl Chloride | 0.05 | 1.85 |
| DB-100 | 0.18 | 6.66 |
| Ionol® (Butylated hydroxytoluene, specifically, 2,6-ditertiary butyl-4-methylphenol) | 0.48 | 17.76 |
| MEMPE | 1.32 | 48.84 |
| PPG-425 | 1.79 | 66.23 |
| PPG-725 | 37.73 | 1396.01 |
| Pluronic® F-108 | 4.00 | 148.00 |
| Total | 100 | 3700 |

**TABLE V**

| (Example 5) | | |
|---|---|---|
| Ingredient | Wt % | Grams |
| Isonate 2143L | 54.45 | 2014.65 |
| Benzoyl Chloride | 0.05 | 1.85 |
| DB-100 | 0.18 | 6.66 |
| Ionol® | 0.48 | 17.76 |
| MEMPE | 1.32 | 48.84 |
| PPG-425 | 20.56 | 760.72 |
| Niax® E-562 | 18.96 | 701.52 |
| Pluronic® F-108 | 4.00 | 148.00 |
| Total | 100 | 3700 |

**TABLE VI**

| (Example 6) | | |
|---|---|---|
| Ingredient | Wt % | Grams |
| Isonate 2143L | 54.44 | 2014.28 |
| Benzoyl Chloride | 0.05 | 1.85 |
| DB-100 | 0.18 | 6.66 |
| Ionol® | 0.48 | 17.76 |
| MEMPE | 1.32 | 48.84 |
| PPG-425 | 20.94 | 774.78 |
| Niax® E-701 | 18.59 | 687.83 |
| Pluronic® F-108 | 4.00 | 148.00 |
| Total | 100 | 3700 |

**TABLE VII**

| (Example 7) | | |
|---|---|---|
| Ingredient | Wt % | Grams |
| Isonate 2143L | 54.44 | 2014.27 |
| Benzoyl Chloride | 0.05 | 1.85 |
| DB-100 | 0.18 | 6.66 |
| Ionol® | 0.48 | 17.76 |
| MEMPE | 1.32 | 48.84 |
| PPG-425 | 20.88 | 772.61 |
| Multranol® 9151 (Mobay Chemical) | 18.65 | 690.01 |
| Pluronic® F-108 | 4.00 | 148.00 |
| Total | 100 | 3700 |

**TABLE VIII**

| (Comparative Example C) | | |
|---|---|---|
| Ingredient | Wt % | Grams |
| Isonate 2143L | 56.42 | 2087.57 |
| Para-toluenesulfonyl chloride | 0.05 | 1.85 |
| DB-100 | 0.18 | 6.66 |
| Ionol® | 0.48 | 17.76 |
| Pluronic® F-108 | 4.00 | 148.00 |
| MEMPE | 1.32 | 48.84 |
| PPG-2025 | 10.60 | 392.19 |
| LG-650 | 5.75 | 212.75 |
| PPG-3025 | 21.20 | 784.38 |
| Total | 100 | 3700 |

**TABLE IX**

| (Example 8) | | |
|---|---|---|
| Ingredient | Wt % | Grams |
| Isonate 2143L | 56.40 | 2086.82 |
| Para-toluenesulfonyl chloride | 0.04 | 1.48 |
| DB-100 | 0.18 | 6.66 |
| Ionol® | 0.48 | 17.76 |
| Pluronic® F-108 | 4.00 | 148.00 |
| MEMPE | 1.32 | 48.84 |
| PPG-2025 | 15.07 | 557.56 |
| LG-650 | 5.75 | 212.75 |
| Niax® E-562 | 16.76 | 620.14 |
| Total | 100 | 3700 |

**TABLE X**

| (Example 9) | | |
|---|---|---|
| Ingredient | Wt % | Grams |
| Isonate 2143L | 56.14 | 2077.10 |
| Para-toluenesulfonyl chloride | 0.04 | 1.48 |
| DB-100 | 0.18 | 6.66 |
| Ionol® | 0.48 | 17.76 |
| Pluronic® F-108 | 4.00 | 148.00 |
| MEMPE | 1.32 | 48.84 |
| PPG-2025 | 16.49 | 609.97 |
| LG-650 | 5.75 | 212.75 |
| Niax® E-701 | 15.61 | 577.44 |
| Total | 100 | 3700 |

**TABLE XI**

| (Example 10) | | |
|---|---|---|
| Ingredient | Wt % | Grams |
| Isonate 2143L | 56.39 | 2086.29 |
| Para-toluenesulfonyl chloride | 0.04 | 1.48 |
| DB-100 | 0.18 | 6.66 |
| Ionol® | 0.48 | 17.76 |
| Pluronic® F-108 | 4.00 | 148.00 |
| MEMPE | 1.32 | 48.84 |
| PPG-2025 | 17.37 | 642.66 |
| LG-650 | 5.75 | 212.75 |
| Multranol® 9151 | 14.47 | 535.55 |
| Total | 100 | 3700 |

After being sealed in the foil pouches for about 3 days, the resin-coated tapes of each of Examples 5-10 and Comparative Examples B and C were removed from their respective pouches and tested for early strength and warm wet strength in accordance with the tests set forth previously. The results are tabulated in Table XII below.

**TABLE XII**

| Example | Polymeric Particle Containing Polyol | Early Strength (newtons/cm) | Warm Wet Strength (newtons/cm) |
|---|---|---|---|
| B | None | 2.74 | 5.87 |
| 5 | Niax E-562 | 4.40 | 7.43 |
| 6 | Niax E-701 | 4.21 | 10.07 |
| 7 | Multranol 9151 | 4.01 | 9.50 |
| C | None | 4.70 | 14.20 |
| 8 | Niax E-562 | 6.56 | 14.47 |
| 9 | Niax E-701 | 6.07 | 19.60 |
| 10 | Multranol 9151 | 5.58 | 17.07 |

As seen in Table XII above, Examples 5, 6, and 7 showed significantly improved early strength over Comparative Example B, and Examples 8, 9, and 10 showed significantly increased early strength over Comparative Example C. With respect to Comparative Example C, the triol weight percent was held constant because, as those skilled in the art know, varying the proportion of low molecular weight triols can have a significant effect on resin properties such as viscosity and ring strength.

Using the resin-coated casting tape rolls from Examples 5, 6, and 7 and Comparative Example B (one roll from each), testing for foaming during cure was carried out using the method described previously under the heading "Foam Reduction Test". Testing was carried out 28 days after coating of the resin onto the scrim. The tapes were aged in foil pouches at about 20°C. Five rolls of the tape from each of the Examples were then refrigerated. One roll from each Example was removed from the refrigerator after 18 days, allowed to warm to about 20°C and tested for foaming. The results are shown in Table XIII below:

**TABLE XIII**

| Example No. | 28 Days @ 20°C (Tspn) | 28 Days @ 20°C plus 18 Days @ 4°C (Tspn) |
|---|---|---|
| 5 | Less than 1 Moderate Persistence | 1 to 2 Moderate Persistence |
| 6 | Less than 1 Least Persistent | Less than 1 Moderate Persistence |
| 7 | 1 to 2 Moderate Persistence | 2 Least Persistent |
| Comparative B | 2 Most Persistent | 3 Most Persistent |

### Examples 11 and 12

In Examples 11 and 12, resin-coated tapes were prepared, cut into individual rolls, and packaged in accordance with the procedure of Example 1, using the ingredients and amounts set forth in Table XIV below, and adding the ingredients in the order listed in Table XIV.

**TABLE XIV**

| Ingredient | Example 11 | | Example 12 | |
|---|---|---|---|---|
| | Wt (kg) | Wt % | Wt (kg) | Wt % |
| Isonate 2143L | 34 | 57.50 | 33.1 | 56.0 |
| Pluronic F-108 | 2.3 | 4.00 | 2.36 | 4.00 |
| DB-100 | 0.10 | 0.18 | 0.10 | 0.18 |
| BHT | 0.28 | 0.48 | 0.28 | 0.48 |
| LG-650 | 3.4 | 5.75 | 0 | 0 |
| PPG-2025 | 12.4 | 21.03 | 0 | 0 |
| MEMPE | 0.68 | 1.15 | 0.78 | 1.32 |
| Niax E-562 | 5.82 | 9.85 | 3.55 | 6.00 |
| Para-toluenesulfonyl chloride | 0.036 | 0.06 | 0.36 | 0.05 |
| PPG-725 | 0 | 0 | 8.90 | 15.06 |
| PPG-425 | 0 | 0 | 10.0 | 16.93 |

### Example 14

An epoxy polymer particle filled polyol is prepared in accordance with the procedure described in Epoxy dispersion polyols: A Novel Polymer-Modified Polyol for Improved Load Bearing and Resiliency (Polyurethane World Congress, 1987).

A bis-phenol A based epoxy resin such as DER-332 (Dow Chemical) is charged with a polyamine such as ethylenediamine, into an ethylene glycol end-capped polypropylene glycol polyol such as Niax® E-351 (Union Carbide). The percent polyethylene glycol in the polyol is preferably less than 20% by weight in order to keep the polyol relatively hydrophobic. Nevertheless, an ethylene glycol capped polyol is preferred in order to increase the amount of grafting of the polyol to the polymer particles, which increases the stability of the dispersion (so called steric stabilization). The epoxy resin:amine equivalent ratio may be from 1:1 to 2:1, but is preferably 1:1. The epoxy resin and amine collectively represent approximately 20% of the total charge (polyol is 80%). The mixture is stirred under high shear. Moderate heat may be necessary. The initial clear solution will turn milky white. The dispersion is stirred until the epoxide group is essentially completely reacted as detected by C13 NMR. The average diameter of the epoxy particles is between 0.2 micron and 10 microns.

This polyol is then be used in place of NIAX E-562 in Example 1 to prepare an orthopedically useful resin.

### Example 15 and Comparative Example D

In Example 15, water-curable resin and resin-coated materials within the scope of the present invention were prepared following the same procedure as set forth for Example 1 above, except that different polymeric particle containing polyols were employed, and one nonparticle containing polyol was employed (Niax E-351 polyol). The relative proportions of the ingredients of all resins are shown below. All resins were formulated to have a NCO/OH ratio 3.45 and an isocyanate equivalent weight of 375.

Comparative Example D was formulated similarly to Example 1 with the major exception that no polymer particle containing Niax polyol was employed. The resins of both Example 15 and Comparative Example D were prepared in accordance with the procedure of Example 1 using the ingredients and amounts set forth in Table XV below, the ingredients having been added in the order indicated in Table XV.

**TABLE XV**

| Ingredient | Equiv. Weight | D Weight (g) | 15a Weight (g) | 15b Weight (g) | 15c Weight (g) | 15d Weight (g) |
|---|---|---|---|---|---|---|
| Isonate 2143L | 145.00 | 2096.29 | 2096.63 | 2101.08 | 2096.46 | 2096.44 |
| Benzoyl Chloride | 140.60 | 1.85 | 1.25 | 1.85 | 1.85 | 1.85 |
| Pluronic F-108 | 7250.00 | 148.00 | 147.21 | 148.00 | 148.00 | 148.00 |
| DB-100 (Now known as Dow Corning Antifoam 1400) | NA | 6.66 | 6.66 | 6.66 | 6.66 | 6.66 |
| PPG-425 Polyol | 211.00 | 890.19 | 907.24 | 913.95 | 928.47 | 915.15 |
| MEMPE | 129.20 | 48.84 | 48.84 | 48.84 | 48.84 | 48.84 |

| Niax Polyols | Varies, see below: | | | | | |
|---|---|---|---|---|---|---|
| E-351 | 1400.00 | 490.62 | 0 | 0 | 0 | 0 |
| E-562 | 1753.00 | 0 | 473.81 | 0 | 0 | 0 |
| E-563 | 1870.00 | 0 | 0 | 461.86 | 0 | 0 |
| E-559 | 2671.00 | 0 | 0 | 0 | 451.96 | 0 |
| E-560 | 2003.00 | 0 | 0 | 0 | 0 | 465.30 |
| Resin viscosity (kcp) | | 97 | 158 | 220 | 138 | 194 |

The different Niax polyols listed above contain varying proportions of polyacrylonitrile as shown in Table XVI.

**TABLE XVI**

| Polymer Polyol | % Styrene in Polymer Particles | % Acrylonitrile in Polymer Particles | % Weight in Total Polymer Particles |
|---|---|---|---|
| Niax E-351 | none | none | none |
| Niax E-562 | 50 | 50 | 20 |
| Niax E-563 | 70 | 30 | 38 |
| Niax E-559 | 70 | 30 | 28 |
| Niax E-560 | 0 | 100 | 21 |

After preparing the resins of Example 15 (a, b, c, and d) and Comparative Example D, the resins were allowed to sit in a sealed container at 23°C for about 24 hours and tested for viscosity using a Brookfield RVT Viscometer with spindle numbers 6 and 7. About 10-12 days later, these resins were individually coated onto the same type of fiberglass scrim and in the same fashion as for Example 1, and individual rolls of each resin-coated material were formed and individually packaged as in Example 1.

Example 15 (a, b, c, and d) and Comparative Example D were tested for foaming in accordance with the procedure previously described. For Example 15a, foaming was less than 1 teaspoon. The consistency of the foam was watery and foam diminished almost as quickly as it was formed. Foaming stopped and all foam disappeared within 45 seconds after starting. Example 15b showed similar results, except foaming stopped and had disappeared approximately one minute after foaming started. Example 15c showed similar results except that foaming stopped even more quickly and had disappeared within 45 seconds; the foam was slightly thicker than for Example 15a. Example 15d exhibited no visual foaming.

For Comparative Example D, the foam was of medium consistency, and although less than one teaspoon of foam produced, there was consistent foaming for a little over one minute. Minimum foaming persisted for a further 20 seconds. Foaming was visibly worse in Comparative Example D than in Examples 15a-d.

The 5 minute ring strength test and the warm wet strength test were then performed on five rolls of the resin-coated tapes of Example 15 and five rolls of the resin-coated tape of Comparative Example D, in accordance with the tests previously set forth in the specification. The average 5 minute strength and average warm wet strength for the Example 15 tapes and Comparative Example D are reported in Table XVII below. Even though the viscosity of the resins containing polymeric particles was higher than the Comparative Example D, the Examples of the invention showed higher 5 minute ring strengths.

**TABLE XVII**

| Example No. | 5 Minute Ring Strength (newtons/cm) | Warm Wet Strength (newtons/cm) |
|---|---|---|
| Comparative D | 9.00 | 18.32 |
| 15a | 12.11 | 19.76 |
| 15b | 9.80 | 42.88 |
| 15c | 11.13 | 28.93 |
| 15d | 10.18 | 28.93 |

### Example 16

This Example combines the resin of this invention with the improved scrim described in United States Patent Application S.N. 376,873, Express Mail Certificate No. B75869812. The resin used is shown in the below Table.

**TABLE XVIII**

| Ingredient | Equivalent Weight | Weight % | Weight (kg) |
|---|---|---|---|
| Isonate 2143L | 144.00 | 57.50 | 74.75 |
| paraToluenesulfonyl Chloride | 191.00 | 0.06 | 0.036 |
| DB 100 (now known as Dow Corning Antifoam 1400) | NA | 0.18 | 0.104 |
| Butylated Hydroxytoluene | 220.40 | 0.48 | 0.28 |
| Pluronic F-108 | 7250.00 | 4.00 | 2.36 |
| MEMPE | 129.2 | 1.15 | 0.68 |
| PPG-2025 | 1002.00 | 21.03 | 12.43 |
| LG-650 | 86.30 | 5.75 | 3.40 |
| NIAX E-562 | 1753.13 | 9.85 | 5.82 |

The resin was coated onto the preferred abraded scrims obtained from Example 6 of the above-referenced patent application by knitting a Scotchcast® 2 fiberglass scrim in the pattern described in that application. The scrim was abraded and coating was carried out in the manner as described in Example 6 of the application but using a large scale coater with a force of 750 newtons between the knurled roller and drive roller and using a deflection of 6 mm. The coating weights of resin were 42.5% on ECDE scrim and 40.0% on ECG scrim.

### Nonorthopedic Applications of the Present Invention

As mentioned previously, although the resin-coated materials of the present invention have exceptional utility as orthopedic casting materials, these resin-coated materials also have a wide variety of other utilities and nonorthopedic uses.

For example, the resin-coated materials of the present invention may be used as protective material to protect the surface of an article or structure from abrasion or corrosion. By way of example only, the resin-coated materials of the present invention may be placed on various structures which are exposed to salt water, for example, oil rigs and ships, so as to protect the underlying structure from salt water corrosion. Similarly, the resin-coated materials may be used on the surfaces of articles or structures which are subject to wear or abrasion. Again, by way of example only, the resin-coated materials may be used to protect dock pilings or other support structures of a dock from abrasion or wear caused by boats butting up against the dock. As a further example, the resin-coated materials may be used to wrap a utility pole or tree so as to prevent rodents from chewing or otherwise damaging the structure of the utility pole or tree.

It will be appreciated that the foregoing examples are merely illustrative, and are not be to considered as comprehensive or in any way limiting. Indeed, the resin-coated materials of the present invention may be used to protect the surface of virtually any article or structure from abrasion and/or corrosion provided that the resin-coated material is capable of being wrapped around the surface of that article or structure. Advantageously, the reduction of foaming in the water phase and other beneficial properties of the resin-coated materials of the present invention are also beneficial in such other nonorthopedic applications.

The resin-coated materials of the present invention also find utility in reinforcing, sealing, or repairing the surface of an article or structure requiring reinforcing, sealing or repairing. For example, the resin-coated materials of the present invention may be wrapped around a leaky pipe or conduit so as to stop the leak and thereby repair the pipe or conduit. Further, the resin-coated materials may be wrapped around the surface of an article to provide a water-tight seal therearound. In addition, the resin-coated materials may be used to join or couple two conduits or objects together. Moreover, the resin-coated materials of the present invention may be used to reinforce or repair conduits carrying fluids, telecommunications conduits, or electrical conduits. Additionally, the resin-coated materials may be used to reinforce or repair cracked articles, such as the handle of a tool or implement.

Again, it will be appreciated that the foregoing examples are not in any way comprehensive or limiting, and that the resin-coated materials of the present invention may be used to reinforce, seal, or repair virtually any article or structure around which the resin-coated materials may be applied. Again, the reduction of foaming in the water phase and other beneficial properties of the present invention are typically of great benefit in these other nonorthopedic applications.

A more detailed description of nonorthopedic applications of resin-coated materials (which nonorthopedic applications are also possible using the resin-coated materials of the present invention) may be found, for example, in U.S. patent application Serial No. 343,432, filed April 26, 1989 (which application is a continuation of U.S. application Serial No. 009,704, filed February 2, 1987, which is a continuation-in-part of U.S. patent application Serial No. 784,671, filed October 4, 1985 (now U.S. Patent No. 4,667,661)), and U.S. patent application Serial No. 343,432, filed April 26, 1989 is incorporated herein by reference.

The present invention may be embodied in other specific forms without departing from its spirit or essential characteristics. The described embodiments are to be considered in all respects only as illustrative and not restrictive. The scope of the invention is, therefore, indicated by the appended claims rather than by the foregoing description. All changes which come within the meaning and range of equivalency of the claims are to be embraced within their scope.

## Claims

1. An article comprising a fabric sheet and a water-curable isocyanate-functional, polyurethane prepolymer resin coated onto said fabric sheet, said prepolymer resin having a stable dispersion of hydrophobic polymeric particles therein subject to the provisos that either
a) said dispersion is free of oil which is immiscible with the prepolymer resin, or
b) said hydrophobic polymeric particles are made from
i) vinyl monomers (but not vinyl monomers resulting in polyolefins or halogenated polyolefins),
ii) polyurea,
iii) epoxy resin-based polymers, or combinations thereof.

2. An article as claimed in claim 1, wherein said polymeric particles have an average diameter of less than 20 microns.

3. An article as claimed in claim 2, wherein said polymeric particles have an average diameter of greater than 0.01 micron.

4. An article as claimed in claim 2 or claim 3, wherein said polymeric particles have an average diameter of less than 10 microns.

5. An article as claimed in claim 4, wherein said polymeric particles have an average diameter of from 0.3 micron to 5 microns.

6. An article as claimed in any of claims 1 to 5, wherein said polymeric particles comprise polyacrylonitrile.

7. An article as claimed in any of claims 1 to 5, wherein said polymeric particles comprise a copolymer of acrylonitrile and styrene.

8. An article as claimed in any preceding claim, wherein said polymeric particles comprise from 0.5% to 10% by weight of said resin.

9. An article as claimed in any of claims 1 to 8 wherein said water-curable resin is an isocyanate functional, polyurethane prepolymer formed by the reaction of a polyol with a polyisocyanate, and wherein said polymeric particles comprise from 10% to 45% by weight of said polyol.

10. An article as claimed in any preceding claim wherein said fabric sheet comprises fiberglass.

11. An article as claimed in any of claims 1 to 5 which is orthopedic casting material.

12. An orthopedic casting material as claimed in claim 11 wherein said fabric sheet has a plurality of projections on at least one side thereof.

13. An orthopedic casting material as claimed in claim 12 wherein each said projection comprises a bundle of at least 8 filaments and wherein said side of said fabric sheet has from 75 to 1500 projections per gram of fabric sheet.

14. An article as claimed in any preceding claim, wherein said polyurethane prepolymer resin further comprises a tack reducing agent which is hydrophilic.

15. An article as claimed in any preceding claim, wherein said stable dispersion of hydrophobic polymeric particles is obtainable by conducting a polymerisation reaction within a polyol to form said polymeric particles therein and said polymeric particle-containing polyol is then reacted with an isocyanate to form said isocyanate functional, polyurethane prepolymer resin.

## Patentansprüche

1. Artikel, umfassend ein textiles Flächengebilde und ein auf dem textilen Flächengebilde aufgetragenes, wässrig härtbares, Isocyanat-funktionelles Polyurethan-Prepolymerharz, wobei das Prepolymerharz darin eine stabile Dispersion hydrophober Polymerpartikel aufweist, unter der Voraussetzung, daß entweder
(a) die Dispersion frei ist von Öl, das mit dem Prepolymerharz unmischbar ist, oder
(b) die hydrophoben Polymerpartikel hergestellt werden aus:
(i) Vinylmomomeren (jedoch nicht aus Vinylmomomeren, die zu Polyolefinen oder halogenierten Polyolefinen führen):
(ii) Polyharnstoff,
(iii) Polymeren auf der Basis von Epoxidharz; oder Kombinationen davon.

2. Artikel nach Anspruch 1, bei welchem die Polymerpartikel einen mittleren Durchmesser kleiner als 20 Mikrometer haben.

3. Artikel nach Anspruch 2, bei welchem die Polymerpartikel einen mittleren Durchmesser größer als 0,01 Mikrometer haben.

4. Artikel nach Anspruch 2 oder 3, bei welchem die Polymerpartikel einen mittleren Durchmesser kleiner als 10 Mikrometer haben.

5. Artikel nach Anspruch 4, bei welchem die Polymerpartikel einen mittleren Durchmesser von 0,3 ... 5 Mikrometer haben.

6. Artikel nach einem der vorgenannte Ansprüche 1 bis 5, bei welchem die Polymerpartikel Polyacrylnitril umfassen.

7. Artikel nach einem der vorgenannte Ansprüche 1 bis 5, bei welchem die Polymerpartikel ein Copolymer aus Acrylnitril und Styrol umfassen.

8. Artikel nach einem der vorgenannte Ansprüche, bei welchem die Polymerpartikel 0,5 % ... 10 Gewichtsprozent des Harzes ausmachen.

9. Artikel nach einem der vorgenannte Ansprüche 1 bis 8, bei welchem das wässrig härtbare Harz ein Isocyanatfunktionelles Polyurethan-Prepolymer ist, gebildet durch die Reaktion eines Polyols mit einem Polyisocyanat, und bei welchem die Polymerpartikel 10 % ... 45 Gewichtsprozent des Polyols ausmachen.

10. Artikel nach einem der vorgenannte Ansprüche 1 bis 8, bei welchem das textile Flächengebilde Glasfaser aufweist.

11. Artikel nach einem der vorgenannte Ansprüche 1 bis 5, welcher Artikel eine orthopädische Vergießmasse ist.

12. Orthopädische Vergießmasse nach Anspruch 11, bei welchem das textile Flächengebilde auf mindestens einer Seite davon eine Vielzahl von Erhebungen aufweist.

13. Orthopädische Vergießmasse nach Anspruch 12, bei welchem jede dieser Erhebungen ein Bündel von mindestens 8 Filamenten umfaßt und bei welchem diese Seite des textilen Flächengebildes 75 ... 1.500 Erhebungen pro Gramm textiles Flächengebilde aufweist.

14. Artikel nach einem der vorgenannte Ansprüche, bei welchem das Polyurethan-Prepolymerharz ferner ein Mittel zum Harabsetzen des Tacks umfaßt, welches Mittel hydrophil ist.

15. Artikel nach einem der vorgenannte Ansprüche, bei welchem die stabile Dispersion von hydrophoben Polymerpartikeln durch Ausführung einer Polymerisationsreaktion im Inneren eine Polyols erhalten werden kann, um darin die Polymerpartikel zu erzeugen, und bei welchem sodann dieses Polymerpartikel enthaltende Polyol umgesetzt wird mit einem Isocyanat, um das Isocyanat-funktionelle Polyurethan-Prepolymerharz zu erzeugen.

## Revendications

1. Article comprenant une feuille de tissu et une résine de prépolymère d'uréthane à fonctionnalité isocyanate, durcissant à l'eau, appliquée sur ladite feuille de tissu, ladite résine de prépolymère contenant une dispersion stable de particules polymères hydrophobes, avec la condition que
a) ladite dispersion ne contienne pas une huile qui n'est pas miscible avec la résine de prépolymère, ou
b) lesdites particules polymères hydrophobes soient formées de :
i) monomères vinyliques, mais pas des monomères vinyliques conduisant à la formation de polyoléfines ou de polyoléfines halogénées,
ii) polyurée,
iii) polymères à base de résine époxy,
ou de mélanges de ceux-ci.

2. Article selon la revendication 1, dans lequel lesdites particules polymères ont un diamètre moyen inférieur à 20 microns.

3. Article selon la revendication 2, dans lequel lesdites particules polymères ont un diamètre moyen supérieur à 0,01 micron.

4. Article selon la revendication 2 ou la revendication 3, dans lequel lesdites particules polymères ont un diamètre moyen inférieur à 10 microns.

5. Article selon la revendication 4, dans lequel lesdites particules polymères ont un diamètre moyen de 0,3 micron à 5 microns.

6. Article selon l'une quelconque des revendications 1 à 5, dans lequel lesdites particules polymères sont constituées de polyacrylonitrile.

7. Article selon l'une quelconque des revendications 1 à 5, dans lequel lesdites particules polymères sont constituées d'un copolymère d'acrylonitrile et de styrène.

8. Article selon l'une quelconque des revendications précédentes, dans lequel lesdites particules polymères représentent de 0,5 % à 10 % en poids de ladite résine.

9. Article selon l'une quelconque des revendications 1 à 8, dans lequel ladite résine durcissant à l'eau est un prépolymère d'uréthane à fonctionnalité isocyanate formé par réaction d'un polyol avec un polyisocyanate, et dans lequel lesdites particules polymères représentent de 10 % à 45 % en poids dudit polyol.

10. Article selon l'une quelconque des revendications précédentes, dans lequel ladite feuille de tissu comprend des fibres de verre.

11. Article selon l'une quelconque des revendications 1 à 5, qui est un matériau pour moulage orthopédique.

12. Matériau pour moulage orthopédique selon la revendication 11, dans lequel ladite feuille de tissu a une pluralité de parties saillantes sur au moins une face.

13. Matériau pour moulage orthopédique selon la revendication 12, dans lequel chacune desdites parties saillantes comprend un faisceau d au moins 8 filaments, et dans lequel ladite face de ladite feuille de tissu a de 75 à 1 500 parties saillantes par gramme de feuille de tissu.

14. Article selon l'une quelconque des revendications précédentes, dans lequel ladite résine de prépolymère de polyuréthane comprend, en outre, un agent réduisant la pégosité, qui est hydrophile.

15. Article selon l'une quelconque des revendications précédentes, dont ladite dispersion stable de particules polymères hydrophobes peut être obtenue en effectuant une réaction de polymérisation à l'intérieur d'un polyol pour former lesdites particules polymères dans celui-ci, et ensuite, en faisant réagir ledit polyol contenant les particules polymères avec un isocyanate pour former ladite résine de prépolymère de polyuréthane à fonctionnalité isocyanate.
